# Europäisches Patentamt

## European Patent Office

## Office européen des brevets

(19)

(11) Veröffentlichungsnummer: **0 062 749**
**B1**

(12) **EUROPÄISCHE PATENTSCHRIFT**

(45) Veröffentlichungstag der Patentschrift:
**22.05.85**

(51) Int. Cl.⁴: **C 01 B 33/193** // C09C1/30,
C08K3/36, C08L21/00

(21) Anmeldenummer: **82101471.9**

(22) Anmeldetag: **26.02.82**

(54) **Verfahren zur Herstellung von Fällungskieselsäuren.**

(30) Priorität: **10.04.81 DE 3114493**

(43) Veröffentlichungstag der Anmeldung:
**20.10.82 Patentblatt 82/42**

(45) Bekanntmachung des Hinweises auf die Patenterteilung:
**22.05.85 Patentblatt 85/21**

(84) Benannte Vertragsstaaten:
**AT BE CH DE FR GB IT LI NL SE**

(56) Entgegenhaltungen:
**DE - A - 2 344 316**
**DE - A - 2 344 805**
**DE - A - 2 446 038**
**DE - A - 2 522 486**
**DE - B - 1 467 019**
**DE - C - 956 495**
**FR - A - 2 090 963**
**US - A - 4 105 757**

(73) Patentinhaber: **Degussa Aktiengesellschaft,**
**Weissfrauenstrasse 9, D-6000 Frankfurt am Main 1 (DE)**

(72) Erfinder: **Nauroth, Peter, Germanusstrasse 8,**
**D-5047 Wesseling (DE)**
Erfinder: **Kuhlmann, Robert, Paul-Keller-Strasse 24,**
**D-5042 Erftstadt (DE)**
Erfinder: **Türk, Günter, Dr., Liesingstrasse 3,**
**D-6450 Hanau 9 (DE)**
Erfinder: **Becker, Adam, Weser Strasse 17,**
**D-5303 Bron.-Hersel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist eine Reihe von Verfahren bekannt, niedrigstrukturierte Fällungskieselsäuren herzustellen:

Nach der DE-A-23 44 316 wird zunächst eine präpolymerisierte Natriumsilikatlösung hergestellt, indem man 5 - 10 Gew.-% Natriumsulfat der Natriumsilikatlösung zusetzt und danach die Kieselsäure durch Zugabe von Säure ausfällt oder einen Teil der präpolymerisierten Natriumsilikatlösung vorlegt, mit Säure auf einen pH-Wert zwischen 6,5 und 11 einstellt und schliesslich die Kieselsäure durch gleichzeitige Zugabe von Natriumsilikatlösung und Säure bei konstantem pH-Wert ausfällt.

Die DE-A-23 44 805 beschreibt ein Verfahren, bei dem man eine Alkalimetallsilikatlösung bis zum Punkt der beginnenden Ausfällung von Fällungskieselsäure ansäuert, dann die Säurezugabe unterbricht, die Reaktionslösung für eine Zeit bis zu vier Stunden lang altert und dann mit der gleichzeitigen Säure- und Alkalimetallsilikatzufuhr fortfährt, bis das gewünschte Produkt erhalten wird.

Gemäss DE-A-24 46 038 wird eine Herstellmethode beschrieben, wonach in eine Elektrolyt enthaltende Lösungsvorlage von 3 - 15 Gew.-% Natriumsulfat bei einem pH-Wert von 9 und bei 65°C gleichzeitig präpolymerisierte Natriumsilikatlösung mit 7 Gew.-% Elektrolytzusatz und verdünnte (10 - 25 Gew.-%ige) Säure eingegeben wird. Anschliessend wird ein pH-Wert unter 5 eingestellt und bei 77°C zwanzig Minuten lang gealtert.

In der DE-A-25 22 486 wird die Herstellung einer Fällungskieselsäure beschrieben, indem ein Teil (8 - 50%) der insgesamt eingesetzten Alkalisilikatlösung als Vorlage eingesetzt wird, in die Alkalimetallsilikatlösung und Säure gleichzeitig einfliessen, und zwar solange, bis bei Beendigung der Alkalimetallsilikat- und Säurezugabe 20 - 500% der ursprünglich vorgelegten Alkalimetallsilikatlösung umgesetzt worden sind. Danach erfolgt die Absenkung des pH-Wertes unter 6. Es muss mit verdünnter Schwefelsäure (8 - 22 Gew.-%) gearbeitet werden.

Nach dem in der deutschen Patentanmeldung S 35 743 IVa/12i am 3.10.1953 ausgelegt am 19.7.1956 als DE-C-956 495 dargelegten Verfahren wird auf eine 2 Gew.-%ige Dispersion bei 80°C und im pH-Bereich 7 bis 7,5, die aus einer «Leichtkieselsäure» (Herstellung nicht offenbart) und Wasser bereitet wird, mit verdünnter Natriumsilikatlösung (Dichte = 1,152) und verdünnter Salzsäure (2 N) über eine Dauer von 2 Stunden aufgefällt. Anschliessend wird mit Wasser verdünnt. Die erhaltene Kieselsäure zeichnet sich durch ein sehr niedriges Schüttgewicht aus.

Obwohl die oben aufgeführten Herstellverfahren zu niedrigstrukturierten Kieselsäuren führen, haften diesen Herstellmethoden gewisse Nachteile an, die ihre technische Durchführung gemessen an den herkömmlichen Wegen zur Gewinnung von Verstärkerkieselsäuren für Kautschuk relativ umständlich und weniger wirtschaftlich machen. Es sind dies:
— die Einführung eines zusätzlichen Teilschrittes für die Bereitung von Kieselsäure- oder elektrolythaltigen Fällungsvorlagen, von elektrolythaltigen Fällungsreaktanden sowie

— die deutliche Absenkung der Raum-Zeit-Ausbeuten bei der Fällung derartiger Kieselsäuren durch die dabei notwendige Einführung eines Unterbrechungsintervalls (Alterungsschritt), durch den Einsatz verdünnter Reaktionskomponenten und durch das Erfordernis langer Fällzeiten.

Unter diesen Umständen ist es ein betriebstechnisches Bedürfnis, Fällungskieselsäuren mit niedriger Struktur auf einfachem, ökonomischen, optimierten, den Betriebsablauf von Verstärkerfüllstoffproduktionen angepasstem Wege herzustellen.

Gemäss DE-B-14 67 019, ist es bekannt, amorphe Fällungskieselsäuren durch gleichzeitige Zugabe von Säure- und Alkalimetallsilikatlösungen zu einer alkalischen Vorlage unter Beachtung gewisser Verfahrensbedingungen auszufällen. Fällungskieselsäuren, die nach diesem Verfahren erhalten werden, lassen sich als Stoffe mit relativ hoher Struktur (relativ hoher Ölaufnahme), relativ hoher spezifischer Oberfläche, niedrigem Schüttgewicht und niedrigem Feststoffgehalt ihres Filterkuchens einstufen. Sie sind auf Grund ihrer hervorragenden Dispergierbarkeit in Gummimischungen bevorzugte Verstärkerfüllstoffe in der Kautschukindustrie, wo sie zur Herstellung von Gummisohlen, Reifenlaufflächen und technischen Artikeln mit oder ohne Silaneinsatz vielfache Verwendung finden. Als verstärkende Standardfüllstoffe und als Hauptprodukte einer jeden Füllstofffabrik steht dieser Typ von Fällungskieselsäuren laufend tagein und tagaus zur Verfügung. Dies gilt auch für die Originalfällungssuspension, die als Zwischenprodukt in grossen Suspensionslagertanks lagert, ehe sie den Filterpressen zur Abtrennung des Elektrolyten zugeführt wird.

Es hat sich nun überraschend gezeigt, dass eine gemäss DE-AS 14 67 019 gewonnene Originalfällungskieselsäuresuspension als billiges Ausgangsprodukt für die Gewinnung von Fällungskieselsäuren mit niedriger Struktur, hoher Härte, und dies selbst bei grosser Feinheit, verwendet werden kann, wenn bestimmte Massnahmen bei der sich anschliessenden Modifizierung und Weiterbearbeitung befolgt werden. Auf diese Weise ist es möglich, die betriebstechnisch umständliche, zusätzliche, häufig auch unwirtschaftliche Zubereitung von Salzlösungen, die als Fällungsvorlage dienen, die ökonomisch völlig untragbare Redispergierung von bereits getrockneten Fällungskieselsäurepulvern zu Kieselsäuredispersionen als Fällungsvorlage, sowie die Bereitstellung von elektrolythaltigen Alkalimetallsilikat- oder Säurelösungen gänzlich zu vermeiden und somit die bestehenden Verfahren des Standes der Technik erheblich zu vereinfachen und zu verbilligen. Dies gilt auch für den anderen Nachteil der herabgesetzten Raum-Zeit-Ausbeute, da das erfindungsgemässe Verfahren ohne Unterbrechungsintervalle mit relativ kurzen Fällzeiten und konzentrierten Reaktionskomponenten auskommt.

Gegenstand der Erfindung ist ein Verfahren zur Herstellung von Fällungskieselsäuren, die je nach ihrem Vermahlungsgrad durch folgende Stoffdaten gekennzeichnet sind:

| | | Pendelmühle | Strahlmühle |
|---|---|---|---|
| BET-Oberfläche nach DIN 66 131 | m²/g | 15 - 110 | 15 - 110 |
| Stampfgewicht nach DIN 53 194 | g/l | 150 - 750 | 90 -.650 |
| Cu-Abrieb in 10%iger Glyzerindispersion | mg | 5 - 30 | 5 - 30 |
| Hellbezugswert A nach DIN 55 921 | % | 86 - 96 | 90 - 96 |
| Teilchengrössenverteilungskurve gemäss Fig. 1 bzw. Fig. 2 nach Coulter Counter | | | |
| «ALPINE-Siebrückstand» > 63 µm | Gew.-% | < 1,5 | < 0,1 |
| Viskosität in 30%iger Glyzerin- -Wasser-Dispersion (1:1) (Brookfield RTV Sp5) | mPas | 30 000 bis 60 000 | |

Die erfindungsgemäss hergestellten Fällungskieselsäuren weisen die folgenden vorteilhaften Eigenschaften auf:

Die hohen Stampfgewichte, die das Dreifache des Wertes der Stampfgewichte der bekannten Fällungskieselsäuren für die Kautschukverstärkung aufweisen, erniedrigen nachhaltig den Aufwand für Verpackungsmaterial, Abfüllung und Transport sowie Lagerung. Bei der Einarbeitung schätzt der Verbraucher die relativ kleinen Verarbeitungsvolumina.

Der hohe, genau einstellbare Cu-Abriebwert gestattet den Einsatz als Schleifmittel und Putzmittel. Dabei zeigt sich überraschend, dass die Abrasivität — nicht wie üblich — mit zunehmender Feinteiligkeit ein und desselben Kieselsäurebasismaterials nachhaltig abnimmt, so dass die kombinierte Eigenschaft Abrasivität und hohe Feinheit diese Kieselsäure zu einem wertvollen Zahnpastenfüllstoff macht. Der Erhalt der Abriebwirkung bei gleichzeitig hoher Feinteiligkeit ermöglicht somit die Anwendung von Strahlmühlen (Luft- bzw. Dampfstrahlmühlen), die infolge des Prinzips der überwiegenden gegenseitigen Kornzerkleinerung den Wandabrieb in der Mahlvorrichtung vermeidet und somit zu helleren, reineren Endprodukten führt als die für die mit mechanischen Prallwerkzeugen ausgerüsteten Mühlen liefern können.

Besonders interessant für die Anwendung der erfindungsgemässen Fällungskieselsäure als Putzkörper in Zahncremes ist die einmalige Kombination von Abrasiv- und Verdickungswirkung. Diese kombinierte Eigenschaft, die nur bei der strahlvermahlenen, feinteiligen Variante beobachtet wird, erlaubt es der kosmetischen Industrie, mit einer einzigen Kieselsäurekomponente und meistens auch mit geringeren Mengen auszukommen.

Gegenstand der Erfindung ist ein Verfahren zur Herstellung der o.a. Fällungskieselsäuren mit je nach ihrem Vermahlungsgrad folgenden physikalisch-chemischen Kenndaten:

| | | Pendelmühle | Strahlmühle |
|---|---|---|---|
| BET-Oberfläche nach DIN 66 131 | m²/g | 15 - 110 | 15 - 110 |
| Stampfgewicht nach DIN 53 194 | g/l | 150 - 750 | 90 - 650 |
| Cu-Abrieb in 10%iger Glyzerindispersion | mg | 5 - 30 | 5 - 30 |
| Hellbezugswert A nach DIN 55 921 | % | 86 - 96 | 90 - 96 |
| Teilchengrössenverteilungskurve gemäss Fig. 1 bzw. Fig. 2 nach Coulter Counter | | | |
| «ALPINE-Siebrückstand» > 63 µm | Gew.-% | < 1,5 | < 0,1 |
| Viskosität in 30%iger Glyzerin- -Wasser-Dispersion (1:1) (Brookfield RTV Sp5) | mPas | 30 000 - 60 000 | |

welches dadurch gekennzeichnet ist, dass man eine Originalfällungskieselsäuresuspension, die durch Fällung der Kieselsäure in einer vorgelegten Alkalisilikatlösung mit einer Konzentration von etwa 5 - 25 g $SiO_2$ je Liter Lösung mit einer Säure- und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derart hergestellt wird, dass die Viskosität des Reaktionsmediums in einem Zeitraum von mindestens 30% der gesamten Fälldauer gleichmässig niedrig und der pH-Wert zwischen 10 und 12 gehalten wird, und die Zugabe der Reaktionsteilnehmer erst dann beendet wird, wenn die Viskosität nach Durchlaufen eines Maximums auf einen weniger als 100% über der Anfangsviskosität liegenden Wert gesunken ist, mit heissem Wasser auf einen Fällungskieselsäuregehalt von 10 - 30 g/l und Natriumsulfatgehalt von 6 - 20 g $Na_2SO_4$/l verdünnt, auf 85 bis 95°C erwärmt, den pH-Wert mit Schwefelsäure auf 7 bis 9 einstellt und unter Konstanthalten dieses pH-Wertes durch gleichzeitigen Zulauf von Alkalimetallsilikatlösung, Schwefelsäure und gegebenenfalls heissem Wasser über eine Fälldauer von 15 bis 180 Minuten eine Fällungskieselsäureendkonzentration von 40 bis 80 g/l einstellt, die Suspension mit konzentrierter Schwefelsäure auf einen pH-Wert unter 7 ansäuert, die Fällungskieselsäure mit Hilfe einer Filterpresse aus der Suspension abtrennt, auswäscht, trocknet und mittels einer Pendelmühle oder Strahlmühle vermahlt.

In einer bevorzugten Ausführungsform kann eine Originalfällungskieselsäuresuspension verwendet werden, die während ihrer Herstellungsphase gemäss DE-B-14 67 019 intensiv geschert wird. Das ist immer dann von Vorteil, wenn besonders hohe Schüttgewichte und hohe Cu-Abriebwerte erzielt werden sollen. In einer besonderen Ausführungsform der Erfindung kann man die Scherung gemäss der DE-C-17 67 332 durchführen.

Die eingesetzte Alkalimetallsilikatlösung kann einen Gewichtsmodul von $SiO_2$ : $Na_2O$ = 2 bis 4 aufweisen. In einer bevorzugten Ausführungsform der Erfindung kann die Alkalimetallsilikatlösung eine Konzentration von 8,0 Gew.-% $Na_2O$ und 26,8 Gew.-% $SiO_2$ aufweisen. Dies entspricht einem Gewichtsmodul von $SiO_2$ : $Na_2O$ = 3,46.

Bei dem gleichzeitigen Zulauf von Alkalisilikatlösung, Schwefelsäure und gegebenenfalls heissem Wasser kann man pro 10 l Fällungskieselsäuresuspension 0,5 bis 10 l Alkalisilikatlösung mit einer Dichte von 1,353, Modul $SiO_2$ : $Na_2O$ = 3,46 pro Stunde, 0,05 bis 1 l Schwefelsäure (96%ig) pro Stunde und bis zu 10 l heisses Wasser pro Stunde zugeben.

Die Konzentration der eingesetzten Schwefelsäure kann bis zu 96 Gew.-% betragen. In einer bevorzugten Ausführungsform kann die Konzentration der Schwefelsäure 50 Gew.-% betragen.

Das Verfahren zur Herstellung der erfindungsgemässen Fällungskieselsäure weist folgende Vorteile auf:
— Der hohe Feststoffgehalt im Filterkuchen bis zu 50 Gew.-% reduziert den Energiebedarf des Trocknungsprozesses und damit die Trocknungskosten auf etwa 25% der Werte der für die im Kautschuksektor verwendeten Verstärkerkieselsäuren,
— der hohe Feststoffgehalt im Filterkuchen bis zu 50 Gew.-% verdoppelt die Filterpressenkapazität,
— die relativ niedrige spezifische Oberfläche der Fällungskieselsäure mit niedriger Struktur führt zu deutlich herabgesetzten Waschzeiten und damit zu Einsparung an Waschwasser und weiterer Filterpressenkapazität und
— es besteht die Möglichkeit, die wichtigsten Stoffdaten der erfindungsgemässen Kieselsäure gezielt einstellen zu können.

Die erfindungsgemässe Fällungskieselsäure und das Verfahren zu ihrer Herstellung werden anhand der folgenden Beispiele näher erläutert und beschrieben:

### Beispiel 1 (Vergleichsbeispiel)

Das Beispiel beschreibt die Herstellung einer Fällungskieselsäure gemäss DE-B-14 67 019.

In einem gummierten 120 l-Fällgefäss werden 73 l heissen Wassers und 5,25 l Natriumsilikatlösung (Dichte: 1,353 g/ml; Modul: $SiO_2$ : $Na_2O$ = 3,46) unter Rühren auf 85°C erhitzt. In diese alkalische Fällungsvorlage werden während der folgenden 90 Minuten unter Rühren und Aufrechterhaltung der Temperatur von 85°C gleichzeitig Natriumsilikatlösung (Dichte = 1,353 g/ml; Modul: $SiO_2$ : $Na_2O$ = 3,46) mit 11 l/h und konzentrierte Schwefelsäure (96%ig) mit 0,965 l/h dosiert.

Danach wird die Fällungskieselsäuresuspension mit konzentrierter Schwefelsäure (96%ig) auf einen pH-Wert von 8,5 eingestellt, was durch einen mehrere Minuten lang andauernden Säurezufluss mit 1,25 l/h geschieht. Die auf diese Weise erhaltene Fällungskieselsäuresuspension hat einen Feststoffgehalt von ca. 85 g/l. Ihr $Na_2SO_4$-Gehalt liegt bei ca. 55 g/l. Sie wird als sogenannte «Originalfällungskieselsäuresuspension» für die Herstellung der in den Folgebeispielen beschriebenen erfindungsgemässen Fällungskieselsäuren eingesetzt.

Die Fällungskieselsäuresuspension wird im weiteren Verlauf mit konzentrierter Schwefelsäure (96%ig) auf einen pH-Wert von 3,5 angesäuert, die Fällungskieselsäure mittels einer Laborfilterpresse aus der Suspension abgetrennt, der Filterkuchen mit Wasser gewaschen, bei 110 - 120°C getrocknet und mit einer Laborstiftmühle vermahlen. Die physikalisch-chemischen Daten sind in Tabelle I aufgeführt.

### Beispiel 2

Die «Originalfällungskieselsäuresuspension» des Beispiels 1 wird mit soviel Wasser verdünnt, dass der Fällungskieselsäuregehalt 20 g/l und der $Na_2SO_4$-Gehalt 13 g/l beträgt.

10,5 l dieser Suspension werden in einem gummierten 30 l-Fällgefäss unter Rühren auf 85°C erhitzt. Unter Aufrechterhaltung dieser Temperatur und eines pH-Wertes von 8,5 werden für die Dauer von 30 Minuten gleichzeitig Wasserglaslösung (8,0% $Na_2O$ und 26,8% $SiO_2$ Dichte: 1,353 g/ml;

Modul $SiO_2$:$Na_2O$ = 3,46) mit einer Geschwindigkeit von 92,4 ml/min., Schwefelsäure (50%ig) mit einer Geschwindigkeit von 19,6 ml/min. und Wasser mit einer Geschwindigkeit von 185,4 ml/min. zur Fällungskieselsäuresuspension gegeben. Die Fällungskieselsäuresuspension wird anschliessend mit Schwefelsäure (50%ig) auf einen pH-Wert von ca. 3,5 eingestellt. Nach beendeter Fällzeit beträgt der Fällungskieselsäuregehalt der Suspension 64 g/l. Die erhaltene Kieselsäure wird mittels einer Laborfilterpresse aus der Suspension abgetrennt, der Filterkuchen mit Wasser salzarm gewaschen, bei 110 - - 120°C getrocknet und mit einer Laborstiftmühle vermahlen. Die Kenndaten sind in Tabelle I aufgeführt.

### Beispiel 3

Es wird wie in Beispiel 2 verfahren, jedoch wird die gleichzeitige Dosierung der Komponenten Wasserglas, Schwefelsäure und Wasser halbiert. Durch diese Massnahme lässt sich im Vergleich zu Beispiel 2 das Stampfgewicht praktisch verdoppeln, während die Abrasivität deutlich abfällt. Der Fällungskieselsäuregehalt der Suspension beträgt 49 g/l. Die Kenndaten sind in Tabelle I aufgeführt.

### Beispiel 4

Es wird wie in Beispiel 3 verfahren, jedoch wird die Fälldauer von 30 auf 60 Minuten ausgedehnt. Der Fällungskieselsäuregehalt wird mit 64 g/l bestimmt. Im Vergleich zu den in den Beispielen 2 und 3 erhaltenen Fällungskieselsäuren liegt das Stampfgewicht deutlich höher. Dies gilt auch für den Cu-Abrieb. Die Kenndaten sind in der Tabelle I aufgeführt.

### Beispiel 5

Es wird gemäss Beispiel 4 verfahren, jedoch wird die Fälldauer von 60 auf 120 Minuten gesteigert. Parallel dazu wird die gleichzeitige Dosierung der Komponenten Wasserglas, Schwefelsäure und Wasser halbiert. Die Dosierung lautet: Wasserglas: 23,1 ml/min., Schwefelsäure: 4,9 ml/min., Wasser: 46,35 ml/min. Der Fällungskieselsäuregehalt stellt sich auf 64 g/l ein. Das Stampfgewicht und die Abriebwerte liegen nochmals höher. Die Kenndaten sind in Tabelle I aufgeführt.

### Beispiel 6

Es wird gemäss Beispiel 4 verfahren; unterschiedlich zu Beispiel 4 wird jedoch die «Originalfällungskieselsäuresuspension» aus Beispiel 1 nur auf 30 g Fällungskieselsäuregehalt/l und etwa 20 g $Na_2SO_4$/l verdünnt. 10,5 l dieser Suspension dienen für die weitere Fällung. Der sich einstellende Fällungskieselsäuregehalt beträgt 71 g/l. Das Stampfgewicht und der Abrieb nehmen im Vergleich zu den Daten der Fällungskieselsäure gemäss Beispiel 4 geringfügig ab. Die Kenndaten sind in Tabelle I aufgeführt.

### Beispiel 7

Es wird gemäss Beispiel 4 verfahren. Unterschiedlich dazu wird jedoch die sogenannte «Originalfällungskieselsäuresuspension» gemäss Beispiel 1 durch zusätzliches Wasser auf 13 g Fällungskieselsäuregehalt/l und 8,5 g $Na_2SO_4$/l verdünnt. Dafür wird auf die Dosierung von 92,7 ml Wasser/min. während der Fällung verzichtet. Es stellt sich ein Fällungskieselsäuregehalt von 64 g/l ein. Die Kenndaten der erhaltenen Fällungskieselsäure sind in Tabelle I aufgeführt.

### Beispiel 8

Es wird gemäss Beispiel 7 verfahren. Im Unterschied dazu wird die Fällung jedoch bei 95°C durchgeführt und parallel dazu die Fällzeit von 60 auf 45 Minuten verkürzt. Es wird ein Fällungskieselsäuregehalt von 52 g/l erhalten. Die Kenndaten der erhaltenen Fällungskieselsäure sind in der Tabelle I aufgeführt.

### Beispiel 9

Zunächst wird gemäss Beispiel 1 eine «Originalfällungskieselsäuresuspension» mit 80 g Fällungskieselsäuregehalt/l und etwa 52 g $Na_2SO_4$/l sowie mit einem pH-Wert von 8,5 hergestellt. Dabei wird jedoch während der gesamten Fälldauer von 90 Minuten mittels einer Kreiselpumpe, die den Behälterinhalt mehrfach umwälzt, intensiv geschert. Nähere Angaben über die apparative Einrichtung sowie über die Scherbedingungen finden sich in der DE-C-17 67 332 und dort insbesondere in Spalte 8, Zeilen 31 - 68.

Die auf diese Weise hergestellte Originalfällungskieselsäuresuspension wird mit Wasser auf einen Fällungskieselsäuregehalt von 20 g/l und 13 g $Na_2SO_4$/l eingestellt. 10,5 l dieser Suspension dienen als Vorlage für die sich dann anschliessende Fällung, die gemäss Beispiel 3 vorgenommen wird.

Der sich einstellende Fällungskieselsäuregehalt beträgt 49 g/l. Die Kenndaten der Fällungskieselsäure finden sich in Tabelle I. Daraus geht hervor, dass durch die Massnahme des Scherens die Abriebwerte und das Stampfgewicht der Fällungskieselsäure erheblich erhöht werden, wenn man die entsprechenden Werte einer Fällungskieselsäure, die unter Verwendung einer nicht gescherten Originalfällungskieselsäuresuspension hergestellt wird, gegenüberstellt.

### Beispiel 10

Es wird gemäss Beispiel 9 verfahren. Einziger Unterschied ist die von 30 auf 60 Minuten verlängerte Fälldauer. Es resultiert ein Fällungskieselsäuregehalt von 64 g/l. Es wird nochmals eine spürbare Steigerung des Abriebwertes (um 92%) und des Stampfgewichtes (um 24%) erreicht.

### Beispiel 11

Das Beispiel soll die grosstechnische Herstellung der erfindungsgemässen Fällungskieselsäure erläutern:

Zunächst wird in einem 70 m³ mit Balkenrührwerk ausgerüsteten Holzfällbottich nach Massgabe der Rezeptur des Beispiels 1, die den Grössenverhältnissen angepasst wird, eine Originalfällungskieselsäuresuspension mit pH-Wert von 8,5 und einem Fällungskieselsäuregehalt von ca. 85 g/l und ca. 55 g $Na_2SO_4$/l hergestellt. 8 m³ dieser Suspension werden nachfolgend in diesem 70 m³-Holzbottich belassen und mit 26 m³ heissem Wasser zu einer Fällungsvorlage mit ca. 20 g Fällungskieselsäure/l und ca. 13 g Na$_2$SO$_4$/l vermischt und auf 85°C erhitzt. Unter Konstanthalten der Temperatur und des pH-Wertes von 8,5 werden für die Dauer von 45 Minuten gleichzeitig 8,8 m$^3$ Natriumsilikatlösung (Dichte: 1,353 g/ml, Modul SiO$_2$:Na$_2$O = 3,46), 0,90 m$^3$ konzentrierte Schwefelsäure (96%ig) und 19,0 m$^3$ heisses Wasser in die Fällungsvorlage dosiert. Nach vollendeter Fällung wird die Suspension mit konzentrierter Schwefelsäure (96%ig) innerhalb weniger Minuten auf einen pH-Wert von 3,5 eingestellt. Der sich einstellende Fällungskieselsäuregehalt liegt bei ca. 64 g/l.

Zur Weiterverarbeitung wird die Fällungskieselsäure aus der Suspension mittels einer Filterpresse abgetrennt, gewaschen, in einem Etagentrockner getrocknet und auf einer Pendelmühle vermahlen. Die Kenndaten der erhaltenen Fällungskieselsäure sind in Tabelle I aufgeführt.

### Beispiel 12

Die apparative Einrichtung entspricht der von Beispiel 11. Zunächst wird auch, wie dort beschrieben, eine «Originalfällungskieselsäuresuspension» mit einem pH-Wert = 8,5, und einem Fällungskieselsäuregehalt von ca. 85 g/l und ca. 55 g/l Na$_2$SO$_4$ hergestellt.

10 m$^3$ dieser Suspension werden nachfolgend im 70 m$^3$-Holzbottich belassen, mit 27 m$^3$ heissem Wasser zu einer Fällungsvorlage mit ca. 23 g Fällungskieselsäure/l und 15 g Na$_2$SO$_4$/l vermischt und auf 85°C erhitzt. Bei dieser Temperatur sowie unter Einhaltung eines pH-Wertes von 8,5 - 9,0 werden für die Dauer von 80 Minuten gleichzeitig 9,6 m$^3$ Natriumsilikatlösung (Dichte: 1,353 g/ml; Modul: SiO$_2$:Na$_2$O = 3,46) 0,94 m$^3$ konzentrierte Schwefelsäure (96%ig) und 20,0 m$^3$ heisses Wasser zur Vorlage dosiert. Nach beendeter Fällung wird die Suspension mit konzentrierter Schwefelsäure (96%ig) auf einen pH-Wert von 3,5 gebracht. Der Fällungskieselsäuregehalt beträgt 64 g/l.

Zur Weiterverarbeitung wird die Fällungskieselsäure aus der Suspension mittels einer Filterpresse abgetrennt, gewaschen, in einem Etagentrockner getrocknet und auf einen Jet-O-mizer, Type 0808 dampfstrahlvermahlen. Die Mahlleistung beträgt 500 kg/h bei einem Mahldruck von 8,5 bar. Die Kenndaten der erhaltenen Fällungskieselsäure sind in Tabelle I aufgeführt.

### Beispiel 13

Es wird gemäss Beispiel 11 verfahren. Einziger Unterschied ist die andersartige Vermahlung mittels einer Dampfstrahlmühle, die gemäss den in Beispiel 12 aufgeführten Bedingungen erfolgt.

### Beispiel 14

Es wird gemäss Beispiel 12 verfahren, jedoch wird die Fälldauer von 80 auf 110 Minuten ausgedehnt. Die dosierten Mengen an konzentrierter Schwefelsäure, an heissem Wasser und Natriumsilikatlösung werden entsprechend angepasst. Der Fällungskieselsäuregehalt beträgt bei beendigter Fällung 64 g/l. Ferner wurden die Dampfstrahlmahlbedingungen geändert, und zwar: Dosierung: 630 kg/h, Mahldruck: 11 bar.

Tabelle I beschreibt die physikalisch-chemischen Kenndaten der gemäss den Beispielen 1 bis 14 hergestellten Fällungskieselsäuren. Die Daten des Beispiels 1 sind die eines Vergleichsbeispiels gemäss dem Stand der Technik, nach dem eine Fällungskieselsäure mit unzureichenden Abriebeigenschaften und niedrigem Stampfgewicht hergestellt wird.

Die physikalisch-chemischen Daten der gemäss den Beispielen 2 bis 10 erhaltenen Fällungskieselsäuren repräsentieren die Produkte von Laborverfahren, während die Daten der Beispiele 11 bis 14 die Stoffdaten der Fällungskieselsäuren von Betriebsverfahren wiedergeben.

Die Stoffdaten spezifische BET-Oberfläche, Stampfgewicht und Hellbezugswert A werden nach DIN-Methoden bestimmt. Bezüglich der Cu-Abriebmessung sowie der Ermittlung des ALPINE-Siebrückstandes > 63 $\mu$ wird auf die nachfolgenden Beschreibungen verwiesen.

### Bestimmung des Cu-Abriebs in 10%iger Glyzerindispersion

a) Herstellung der Glyzerindispersion

153 g Glyzerin (DAB 7; S = 1,26), wasserfrei, werden in einem Polyäthylenbecher (250 ml) eingewogen. Mit dem Spatel werden vorsichtig 17 g Fällungskieselsäure untergemischt. Die Mischung wird anschliessend mit einem Flügelrührer (Durchmesser 5,2 cm) 12 Minuten lang bei 1.500 Upm homogenisiert.

b) Durchführung der Abriebmessung

Die Bestimmung der Abriebmessung erfolgt in dem Abriebtestgerät, welches gemäss den folgenden Druckschriften bekannt ist: (1) Pfrengle: Fette, Seifen, Anstrichmittel, 63 (5) (1961), Seiten 445 bis 451 «Abrasion und Reinigungskraft von Putzkörpern in Zahnpasten» (2) A. RENG, F. DANY; Parfümerie und Kosmetik 59 (2) (1978), Seiten 37 bis 45; «Anwendungstechnische Prüfung von Zahnpasten». Dazu werden die 6 Tröge des Testgerätes mit je 20 ml der homogenen Dispersion beschichtet. Der Abrieb, sen sechs plangeschliffene Nylonbürsten an sechs plangeschliffenen Cu-Blechen (Elektrolyt-Kupfer) in fünf Stunden mit 50.000 Doppelhüben bewirken, wird durch Differenzwägung bestimmt. Bei der Berechnung der Abrasivität werden von den erhaltenen Werten Mittelwerte gebildet. Der Abrieb (Abrasivität) wird in mg Cu angegeben.

### Bestimmung des Siebrückstandes mit dem ALPINE-Luftstrahl-Sieb

Zur Bestimmung des Siebrückstandes wird die Fällungskieselsäure durch ein 500 $\mu$m Sieb abgesiebt, um eventuell vorhandene Entlüftungsknoten zu zerstören. Dann werden 10 g des gesiebten Materials auf ein bestimmtes Luftstrahlsieb gegeben und bei 200 mm Wassersäulenunterdruck ( = 26666,666 Pa) abgesiebt. Fällungskieselsäurepartikel, die sich am Plexiglasdeckel des Siebgerätes absetzen, werden durch einige Schläge auf den Knopf des Siebdeckels abgeklopft. Die Siebung ist beendet, wenn der Rückstand konstant bleibt, was meistens am rieselfähigen Aussehen zu erkennen ist. Zur Sicherheit siebt man dann noch eine Minute. Im allgemeinen dauert der Siebvorgang fünf Minuten. Bei Materialien, die Kornanteile von < 500 μm haben, wird die Probe vorher nicht abgesiebt, sondern direkt auf das Luftstrahlsieb gegeben. Bei eventuell sich bildenden Agglomeraten wird der Siebvorgang kurz unterbrochen und die Agglomerate mit einem Pinsel unter leichtem Druck zerstört. Nach der Siebung wird der Siebrückstand vorsichtig von dem Luftstrahlsieb geklopft und zurückgewogen.

*Berechnung:* Der Siebrückstand wird in Prozenten in Verbindung mit der Maschenweite des Siebes angegeben.

*Geräte:* Alpine Luftstrahlsieb, Labortyp S 200 Luftstrahlsieb mit Siebgewebe nach DIN 4 188.

Prüfung der rheologischen Wirksamkeit von Abrasivkieselsäuren in 30%iger Glyzerin/Wasser-Dispersion (Gemisch 1:1) Brookfield RTV Sp5.

1. *Probensätze*
  60 g Kieselsäure
  70 g Glyzerin wasserfrei DAB 7, Dichte ml/g 1,26
  70 g Wasser dest.
  ————
200 g 30%ige Dispersion bezogen auf Kieselsäure

2. *Durchführung*
Die Abrasivkieselsäuren wurden von Hand in einem 400 ml-Becherglas (breite Form) in das Glyzerin/Wasser-Gemisch mit einem Glasstab eingerührt (1 Min.) und 24 Stunden stehen gelassen. Danach wird die Viskosität gemessen.

3. *Messung*
Die Viskositätsmessung wird im selben Becherglas mit dem Brookfield-Viskosimeter RVT Spindel 5 bei verschiedenen Upm. durchgeführt.

4. *Ausrechnung*
Abgelesener Skalenwert × Faktor = Viskosität in mPas.

*Tabelle I*     Physikalisch-chemische Kenndaten der gemäss Beispiele 1 bis 14 hergestellten Fällungskieselsäuren

| Physikalisch-chemische Kenngrösse | | 1 | 2 | 3 | 4 | 5 | 6 | 7 | 8 | 9 | 10 | 11 | 12 | 13 | 14 |
|---|---|---|---|---|---|---|---|---|---|---|---|---|---|---|---|
| BET-Oberfläche DIN 66 131 | m²/g | 186 | 73 | 78 | 41 | 24 | 40 | 58 | 40 | 48 | 18 | 103 | 70 | 101 | 51 |
| Stampfgewicht DIN 53 194 | g/l | 80 | 98 | 172 | 244 | 345 | 218 | 111 | 210 | 520 | 645 | 270 | 138 | 110 | 156 |
| ALPINE-Siebrückstand > 63 μ | Gew.-% | 1,2 | 0,3 | <0,1 | 0,1 | <0,1 | <0,1 | 0,2 | <0,1 | <0,1 | <0,1 | 1,4 | <0,1 | <0,1 | <0,1 |
| Cu-Abrieb in 10%iger Glyzerindispersion | mg | <1 | 11 | 7 | 23 | 27 | 19 | 16 | 18 | 13 | 25 | 14 | 12 | 8 | 17 |
| Hellbezugswert A DIN 55 921 | % | 96,3 | 96,0 | 95,8 | 91,0 | 87,8 | 92,7 | 94,7 | 93,5 | 93,2 | 89,6 | 94,4 | 95,7 | 96,8 | 94,6 |

**Patentansprüche**

1. Verfahren zur Herstellung einer Fällungskieselsäure mit den folgenden physikalisch-chemischen Kenndaten:

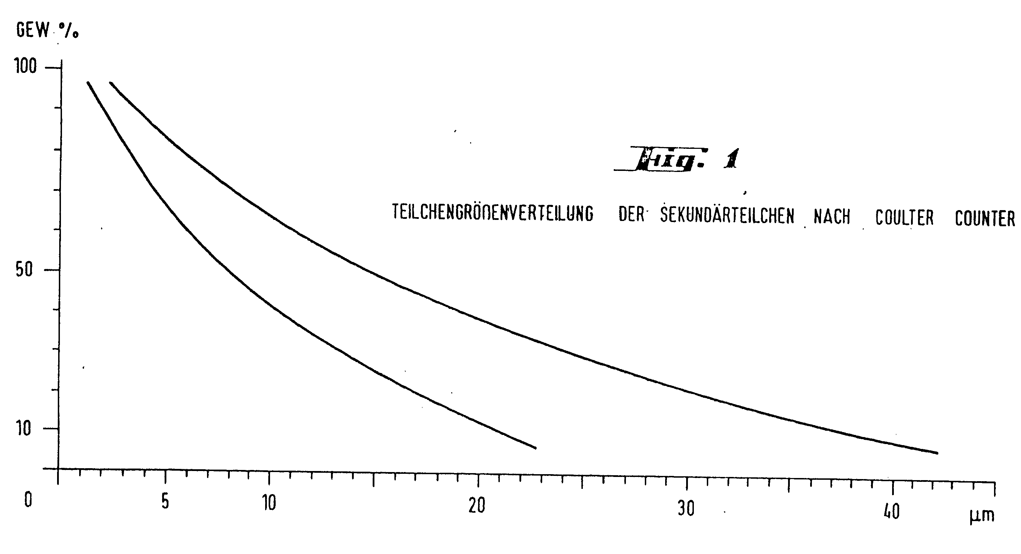

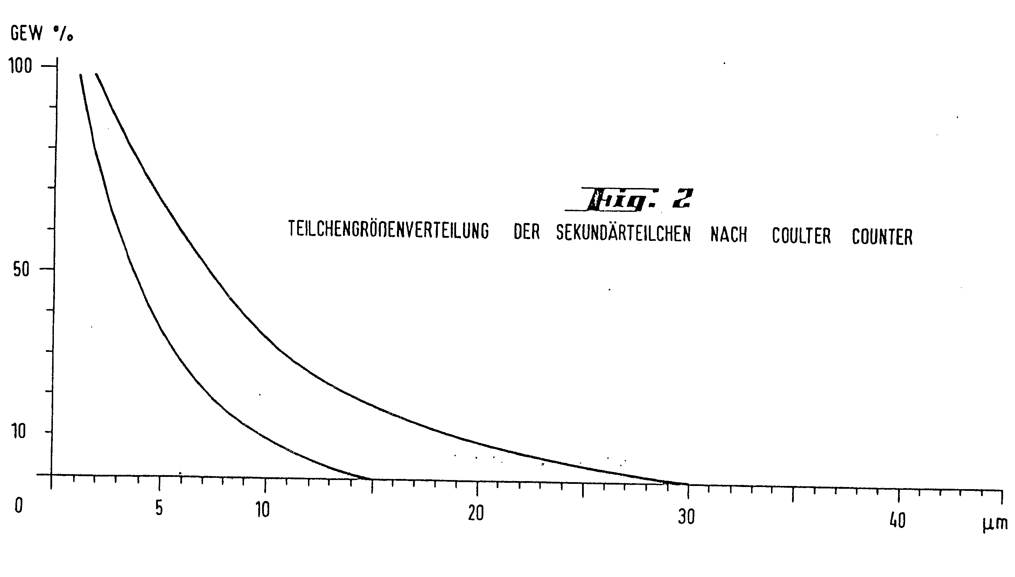

BET-Oberfläche nach DIN 66 131     m²/g     15 - 110
Stampfgewicht nach DIN 53 194     g/l     150 - 750
Cu-Abrieb in 10%iger Glyzerindispersion     mg     5 - 30
Hellbezugswert A nach DIN 55 921     %     86 - 96
Teilchengrössenverteilungskurve der Sekundärteilchen gemäss Fig. 1 nach Coulter Counter
«ALPINE-Siebrückstand» > 63 μm     Gew.-%     < 1,5
Viskosität in 30%iger Glyzerin-Wasser-Dispersion (1:1) (Brookfield RTV Sp5)     mPas     30000 - 60000

dadurch gekennzeichnet, dass man eine Originalfällungskieselsäuresuspension, die durch Fällung der Kieselsäure aus einer vorgelegten Alkalisilikatlösung mit einer Konzentration von etwa 5 - 25 g SiO₂ je Liter Lösung mit einer Säure und Alkalimetallsilikatlösung mit bestimmten Lösungskonzentrationen und bestimmten Zulaufgeschwindigkeiten unter Aufrechterhaltung einer Fälltemperatur im Reaktionsmedium zwischen 80 und 90°C derart hergestellt wird, dass die Viskosität des Reaktionsmediums in einem Zeitraum von mindestens 30% der gesamten Fälldauer gleichmässig niedrig und der pH-Wert zwischen 10 und 12 gehalten wird, und die Zugabe der Reaktionsteilnehmer erst dann beendet wird, wenn die Viskosität nach Durchlaufen eines Maximums auf einen weniger als 100% über der Anfangsviskosität liegenden Wert gesunken ist, mit heissem Wasser auf einen Fällungskieselsäuregehalt von 10 - 30 g/l und Natriumsulfatgehalt von 6 - 20 g Na₂SO₄/l verdünnt, auf 85 - 95°C erwärmt, den pH-Wert mit Schwefelsäure auf 7 bis 9 einstellt und unter Konstanthalten dieses pH-Wertes durch gleichzeitigen Zulauf von Alkalimetallsilikatlösung, Schwefelsäure und gegebenenfalls heissem Wasser über eine Fälldauer von 15 bis 180 Minuten eine Fällungskieselsäureendkonzentration von 40 bis 80 g/l einstellt, die Suspension mit konzentrierter Schwefelsäure auf einen pH-Wert unter 7 ansäuert, die Fällungskieselsäure mit Hilfe einer Filterpresse aus der Suspension abtrennt, auswäscht, trocknet und mittels einer Pendelmühle vermahlt.

2. Verfahren zur Herstellung einer Fällungskieselsäure mit den folgenden physikalisch-chemischen Kenndaten:

| | | |
|---|---|---|
| BET-Oberfläche nach DIN 66 131 | $m^2$/g | 15 - 110 |
| Stampfgewicht nach DIN 53 194 | g/l | 90 - 650 |
| Cu-Abrieb in 10%iger Glyzerindispersion | mg | 5 - 30 |
| Hellbezugswert A nach DIN 55 921 | % | 90 - 96 |
| Teilchengrössenverteilungskurve der Sekundärteilchen gemäss Fig. 2 nach Coulter Counter «ALPINE-Siebrückstand» > 63 $\mu$m | Gew.-% | < 0,1 |
| Viskosität in 30%iger Glyzerin-Wasser-Dispersion (1:1) (Brookfield RTV Sp5) | mPas | 30000 - 60000 |

dadurch gekennzeichnet, dass man eine Originalfällungskieselsäuresuspension, die durch Fällung der Kieselsäure aus einer vorgelegten Alkalisilikatlösung mit einer Konzentration von etwa 5 - 25 g $SiO_2$ je Liter Lösung mit einer Säure- und Alkalisilikatlösung mit bestimmten Lösungskonzentrationen und bestimmten Zulaufgeschwindigkeiten unter Aufrechterhaltung einer Fälltemperatur, im Reaktionsmedium zwischen 80 und 90°C derart hergestellt wird, dass die Viskosität des Reaktionsmediums in einem Zeitraum von mindestens 30% der gesamten Fälldauer gleichmässig niedrig und der pH-Wert zwischen 10 und 12 gehalten wird, und die Zugabe der Reaktionsteilnehmer erst dann beendet wird, wenn die Viskosität nach Durchlaufen eines Maximums auf einen weniger als 100% über der Anfangsviskosität liegenden Wert gesunken ist, mit heissem Wasser auf einen Fällungskieselsäuregehalt von 10 - 30 g/l und Natriumsulfatgehalt von 6 - 20 g $Na_2SO_4$/l verdünnt, auf 85 - 95°C erwärmt, den pH-Wert mit Schwefelsäure auf 7 bis 9 einstellt und unter Konstanthalten dieses pH-Wertes durch gleichzeitigen Zulauf von Alkalisilikatlösung, Schwefelsäure und heissem Wasser über eine Fälldauer von 15 bis 180 Minuten eine Fällungskieselsäureendkonzentration von 40 - 80 g/l einstellt, die Suspension mit konzentrierter Schwefelsäure auf einen pH-Wert unter 7 ansäuert, die Fällungskieselsäure mit Hilfe einer Filterpresse aus der Suspension abtrennt, auswäscht, trocknet und mittels einer Strahlmühle vermahlt.

3. Verfahren nach den Ansprüchen 1 und 2, dadurch gekennzeichnet, dass die hergestellte Originalfällungskieselsäuresuspension bis zum Zeitpunkt ihrer Verdünnung mit heissem Wasser während der gesamten Phase ihrer Herstellung intensiv geschert wird.

**Claims**

1. A process for the production of a precipitated silica having the following physico-chemical characteristics:

| | | |
|---|---|---|
| BET surface area according to DIN 66 131 | $m^2$/g | 15 - 110 |
| Tamped density according to DIN 53 194 | g/l | 150 - 750 |
| Cu-grindings in 10% glycerine dispersion | mg | 5 - 30 |
| Reference brightness value A according to DIN 55 921 | % | 86 - 96 |
| Particle size distribution curve of secondary particles according to the Coulter Counter as shown in Fig. 1 «Alpine sieve residue» > 63 $\mu$m | % by weight | < 1.5 |
| Viscosity in 30% glycerine/water dispersion (1:1) (Brookfield RTV Sp5) | mPas | 30000 - 60000 |

characterised in that an original precipitated silica suspension produced by precipitation of the silica from a starting alkali silicate solution with a concentration of about 5 to 25 g of $SiO_2$ per litre of solution with an acid and with alkali metal silicate solution having specific solution concentrations and specific feed rates while maintaining a precipitation temperature in the reaction medium between 80 and 90°C in such a way that the viscosity of the reaction medium is kept uniformly low over a period of at least 30% of the total precipitation period and the pH is kept between 10 and 12, and the addition of the reactants is stopped only once the viscosity, after passing through a maximum has fallen to a value less than 100% above the starting viscosity, is diluted with hot water to a precipitated silica content of 10 to 30 g/l and a sodium sulphate content of 6 to 20 g $Na_2SO_4$/l, is heated to 85 to 95°C, the pH is adjusted to 7 to 9 using sulphuric acid and a final precipitated silica concentration of 40 to 80 g/l is adjusted while maintaining this pH by simultaneous supply of alkali metal silicate solution, sulphuric acid and optionally hot water over a precipitation period of 15 to 180 minutes, the suspension is acidified to a pH below 7 using concentrated sulphuric acid, the precipitated silica is separated from the suspension by means of a filter press, is washed out, dried and ground using a vibrating mill.

2. A process for the production of a precipitated silica having the following physico-chemical characteristics:

| | | |
|---|---|---|
| BET surface area according to DIN 66 131 | $m^2$/g | 15 - 110 |
| Tamped density according to DIN 53 194 | g/l | 90 - 650 |
| Cu-grindings in 10% glycerine dispersion | mg | 5 - 30 |
| Reference brightness value A according to DIN 55 921 | % | 90 - 96 |
| Particle size distribution curve of secondary particles according to the Coulter Counter as shown in Fig. 2 «Alpine sieve residue» > 63 $\mu$m | % by weight | < 0.1 |
| Viscosity in 30% glycerine/water dispersion (1:1) (Brookfield RTV Sp5) | mPas | 30000 - 60000 |

characterised in that an original precipitated silica suspension produced by precipitation of the silica from a starting alkali silicate solution with a concentration of about 5 to 25 g of $SiO_2$ per litre of solution with an acid and alkali silicate solution having specific solution concentrations and specific feed rates while maintaining a precipitation temperature in the reaction medium of between 80 and 90°C in such a way that the viscosity of the reaction medium is kept uniformly low over a period of at least 30% of the total precipitation period and the pH is kept between 10 and 12, and the addition of the reactants is stopped only once the viscosity, after passing through a maximum, has fallen to a value less than 100% above the starting viscosity, is diluted with hot water to a precipitated silica content of 10 to 30 g/l and a sodium sulphate content of 6 to 20 g $Na_2SO_4$/l, is heated to 85 to 95°C, the pH is adjusted to 7 to 9 using sulphuric acid and a final precipitated silica concentration of 40 to 80 g/l is adjusted while maintaining this pH by simultaneous supply of alkali metal silicate solution, sulphuric acid and hot water over a precipitation period of 15 to 180 minutes, the suspension is acidified to a pH below 7 using concentrated sulphuric acid, the precipitated silica is separated from the suspension using a filter press, is washed out, dried and ground using a vibrating mill.

3. A process according to claims 1 and 2, characterised in that the original precipitated silica suspension produced is intensively sheared throughout its production phase until its moment of dilution with hot water.

**Revendications**

1. Procédé pour l'obtention d'une silice précipitée ayant les caractéristiques physico-chimiques suivantes:

| | | |
|---|---|---|
| Surface BET selon DIN 66 131 | m²/g | 15 - 110 |
| Densité tassée selon DIN 53 194 | g/l | 150 - 750 |
| Abrasion de Cu dans une dispersion de glycérine à 10% | mg | 5 - 30 |
| Valeur relative de luminosité selon DIN 55 921 | % | 86 - 96 |
| Courbe de répartition granulométrique des particules secondaires selon la figure 1, par compteur Coulter «Refus au tamis Alpine» 63 μ | % en poids | < 1,5 |
| Viscosité dans une dispersion glycérine - eau à 30% (1:1) (Brookfield RTV Sp5) | mPas | 30000 - 60000 |

procédé caractérisé en ce qu'une suspension de silice précipitée initiale est préparée par précipitation de la silice à partir d'une solution de silicate alcalin, préparée elle-même avec une concentration d'environ 5 à 25 g de $SiO_2$ par litre de solution, avec un acide et une solution de silicate de métal alcalin, avec des concentrations de solution déterminées et des vitesses d'introduction déterminées, en maintenant une température de précipitation dans le milieu réactionnel comprise entre 80 et 90°C, de telle façon que la viscosité du milieu réactionnel soit uniformément faible pendant une durée d'au moins 30% de la durée de précipitation totale et que le pH soit compris entre 10 et 12, et l'addition des partenaires réactionnels se termine seulement lorsque la viscosité, après passage par un maximum, est descendue à une valeur inférieure à 100% au-dessus de la viscosité initiale, que cette suspension initiale est diluée avec de l'eau chaude de façon à obtenir une teneur en silice précipitée de 10 - 30 g/l et une teneur en sulfate de sodium de 6 - 20 g de $Na_2SO_4$/l, chauffée à 85 à 95°C, le pH est ajusté avec de l'acide sulfurique à une valeur de 7 à 9 et, en maintenant ce pH à une valeur constante, qu'il est établi, par introduction simultanée d'une solution de silicate de métal alcalin, d'acide sulfurique et évetuellement d'eau chaude, pendant une durée de précipitation de 15 à 180 minutes, une concentration finale de silice précipitée de 40 à 80 g/l, la suspension est acidifiée avec de l'acide sulfurique concentré à un pH inférieur à 7, et que la silice précipitée est séparée de la suspension à l'aide d'un filtre-presse, lavée, séchée et broyée à l'aide d'un broyeur à cylindres.

2. Procédé pour l'obtention d'une silice précipitée ayant les caractéristiques physico-chimiques suivantes:

| | | |
|---|---|---|
| Surface BET selon DIN 66 131 | m²/g | 15 - 110 |
| Densité tassée selon DIN 53 194 | g/l | 90 - 650 |
| Abrasion de Cu dans une dispersion de glycérine à 10% | mg | 5 - 30 |
| Valeur relative de luminosité selon DIN 55 921 | % | 90 - 96 |
| Courbe de répartition granulométrique des particules secondaires selon la figure 2, par compteur Coulter «Refus au tamis Alpine» 63 μ | % en poids | < 0,1 |
| Viscosité dans une dispersion glycérine - eau à 30% (1:1) (Brookfield RTV Sp5) | mPas | 30000 - 60000 |

procédé caractérisé en ce qu'une suspension de silice précipitée initiale est préparée par précipitation de la silice à partir d'une solution de silicate alcalin, préparée elle-même, avec une concentration d'environ 5 à 25 g de $SiO_2$ par litre de solution, avec un acide et une solution de silicate de métal alcalin, avec des concentrations de solution déterminées et des vitesses d'introduction déterminées, en maintenant une température de précipitation dans le milieu réactionnel comprise entre 80 et 90°C, de telle façon que la viscosité du milieu réactionnel soit uniformément faible pendant une durée d'au moins 30% de la durée de précipitation totale, et que le pH soit compris entre 10 et 12, et l'addition des partenaires réactionnels se termine seulement lorsque la viscosité, après passage par un maximum, est descendue à une valeur inférieure à 100% au-dessus de la viscosité initiale, que cette suspension initiale est diluée avec de l'eau chaude de façon à obtenir une teneur en silice précipitée de 10 - 30 g/l et une teneur en sulfate de sodium de 6 - 20 g de $Na_2SO_4$/l, chauffée à 85 à 95°C, le pH est ajusté avec de l'acide sulfurique à une valeur de 7 à 9 et, en maintenant ce pH à une valeur constante, qu'il est établi, par introduction simultanée d'une solution de silicate de métal alcalin, d'acide sulfurique et éventuellement d'eau chaude, pendant une durée de précipitation de 15 à 180 minutes, une concentration finale de silice précipitée de 40 à 80 g/l, la suspension est acidifiée avec de l'acide sulfurique concentré à un pH inférieur à 7, et que la silice précipitée est séparée de la suspension à l'aide d'un filtre-presse, lavée, séchée et broyée à l'aide d'un broyeur à jet.

3. Procédé selon les revendications 1 et 2, caractérisé en ce que la suspension de silice précipitée initiale préparée est soumise à un cisaillement intense, jusqu'au moment de sa dilution à l'eau chaude, pendant toute la phase de sa préparation.

GEW %

TEILCHENGRÖßENVERTEILUNG  DER SEKUNDÄRTEILCHEN NACH COULTER COUNTER

Fig. 1

0 062 749

GEW %

TEILCHENGRÖßENVERTEILUNG DER SEKUNDÄRTEILCHEN NACH COULTER COUNTER

Fig. 2